# EUROPEAN PATENT APPLICATION

(11) **EP 3 422 003 A1**
(43) Date of publication of application: **02.01.2019**
(21) Application number: 17305810.8
(22) Date of filing: 28.06.2017
(51) Int. Cl.: G01N 33/574

(54) **METHOD FOR DETERMINING IF A PATIENT IS AT RISK OF HAVING OR DEVELOPING A COLORECTAL CANCER (CRC)**

(71) Applicant: Centre Léon Bérard, 69008 Lyon (FR)
(72) Inventor: MARIE, Julien, 69007 Lyon (FR); BAUCHE, David, 21800 Chevigny-Saint-Sauveur (FR)
(74) Representative: Lavoix

(57) **Abstract**

The present invention relates to a method for determining if a patient is at risk of having or developing a colorectal cancer (CRC). It further relates to a method for selecting a patient who will most likely benefit from a preventive treatment with an antibiotic, in particular Vancomycin and/or with injections of recombinant human IL-22 and/or anti- human IL-17A antibody.

## Description

The present invention relates to a method for determining if a patient is at risk of having or developing a colorectal cancer (CRC). It further relates to a method for selecting a patient who will most likely benefit from a preventive treatment with an antibiotic, in particular Vancomycin and/or with injections of recombinant human IL-22 and/or antihuman IL-17A antibody.

### Background of the invention

The human intestine harbors an enormous diversity of microorganisms that far outnumber the eukaryotic cells of the host. Interestingly, this microbiota is particularly abundant in the colon where cancer incidence is the highest of the whole intestine. Over the last decade, many studies have reported that the microbiota can exacerbate the growth of already established tumors, using either APC^{Min} mice that are highly susceptible to gut adenoma, or mice exposed to colonic carcinogens including AOM (Azoxymethane). However, whether the gut microbiota contributes to spontaneous generation of colorectal cancers (CRC) and which mechanisms are in place to counteract such potential threats remain unknown. The mammalian gut is enriched in Transforming Growth Factor beta (TGF-b), produced by various cell-types, including immune cells and epithelial cells.

In the context of the present invention, the inventors show that by measuring in a biopsy or blood sample from a patient the level of expression of Transforming Growth Factor BetaRII receptor (TGFβRII) on Intestinal or blood Group 3 Innate Lymphoid Cells (ILC3) and the intestinal or blood percentage of CD4 T lymphocytes producing Interleukin 17 (IL-17A; or the percentage of ILC3 producing Interleukin 22 (IL-22) and the intestinal or blood percentage of CD4 T lymphocytes producing Interleukin 17 (IL-17A); or the activation of T lymphocytes by the intestinal commensal bacteria; it is possible to determine if a patient is at risk of having or developing a colorectal cancer (CRC)

### Description of the invention

The present invention thus relates to a method for determining if a patient is at risk of having or developing a colorectal cancer (CRC), comprising the step of measuring in a biopsy or blood sample from said patient:
(a) the level of expression of Transforming Growth Factor BetaRII receptor (TGFβRII) on intestinal or blood Group 3 Innate Lymphoid Cells (ILC3) and the intestinal or blood percentage of CD4 T lymphocytes producing Interleukin 17 (IL-17A; or
(b) the percentage of ILC3 producing Interleukin 22 (IL-22) and the Intestinal or blood percentage of CD4 T lymphocytes producing Interleukin 17 (IL-17A); or
(c) the activation of T lymphocytes by the intestinal commensal bacteria.

The method according to the invention has the advantage to be a quick method that does not involve additional invasion for the patient.

In addition, the current methods are based on the presence of blood in the stools corresponding to a clinical sign partly too late as the cancer is already there.

In one embodiment, the method as described above comprises the step of measuring in a biopsy or blood sample from said patient:
(a) the level of ILC3 bearing TGFβRII and the percentage of IL-17 produced by CD4 T cells.

In particular, the ILC3 bearing TGFβRII level is measured through flow-cytometry. More particularly, the level of ILC3 bearing TGFβRII measured through flow-cytometry is obtained after labeling with a detectable antibody binding to TGFβRII.

In particular, the percentage of IL-17 produced by CD4 T cells is measured through flow-cytometry.

More particularly the percentage of IL-17 produced by CD4 T cells measured through flow-cytometry is obtained after labeling with anti-IL-17A antibody.

In one embodiment of the method as described herein, a ratio between the level TGFβRII expression on ILC3 and the percentage of IL-17 producing CD4 T cells below 85 is indicative of a patient at risk of having or developing a CRC. In particular, this ratio indicates a probability to have or develop a CRC of 70%.

In the same manner, a ratio between the level TGFβRII expression on ILC3 and the percentage of IL-17 producing CD4 T cells above 85 indicates a probability to be healthy at 100%.

In another embodiment, the method as described above comprises the step of measuring in a biopsy or blood sample from said patient:
(b) the percentage of IL-22 produced by ILC3 and the percentage of IL-17 produced by CD4 T cells.

In particular, the percentage of IL-22 produced by ILC3 is measured through flow-cytometry.

More particularly, the percentage of IL-22 produced by ILC3 measured through flow-cytometry is obtained after labeling with anti-IL-22 antibody.

In particular, the percentage of IL-17 produced by CD4 T cells is measured through flow-cytometry.

More particularly the percentage of IL-17 produced by CD4 T cells measured through flow-cytometry is obtained after labeling with anti-IL-17A antibody.

In another embodiment of the method as described herein, a ratio between the percentage of ILC3 producing IL-22 and the intestinal or blood percentage of CD4 T lymphocytes producing IL-17A below 0.5 is indicative of a patient at risk of having or developing a CRC.

In the same manner, a ratio between the percentage of ILC3 producing IL-22 and the intestinal or blood percentage of CD4 T lymphocytes producing IL-17A above 0.7 indicates a probability to be healthy at 100%.It is more difficult to take position for a ratio between the percentage of ILC3 producing IL-22 and the intestinal or blood percentage of CD4 T lymphocytes producing IL-17A between 0.5 and 0.7. If the ratio is closer to 0.5, it will be more probable that the patient at risk of having or developing a CRC than if the ratio is closer to 0.7. The window between 0.5. and 0.7 is regarded as non totally predictive, and said type of patient will require a second analysis within the next 6 months.

In still another embodiment, the method as described above comprises the step of measuring in a biopsy or blood sample from said patient:
(c) the activation of T lymphocytes by the intestinal commensal bacteria.

In particular, an activation of T cells by the intestinal commensal bacteria is indicative of a patient at risk of having or developing a CRC.

More particularly, the activation of T cells by the intestinal commensal bacteria is measured by comparing the level of expression of IL-17 and/or IFN-gamma to a control value.

Even more particularly, a level of expression of IL-17 and/or IFN-gamma higher than the control value indicates the activation of T cells by the intestinal commensal bacteria and thus indicates that the patient is at risk of having or developing a CRC.

The present invention further relates to a method for selecting a patient who will most likely benefit from a preventive treatment with an antibiotic, in particular Vancomycin and/or with injections of recombinant human IL-22 and/or human anti-IL-17A antibody, wherein said method comprises determining if a patient is at risk of having or developing a colorectal cancer by the method as above described.

"Colorectal cancer (CRC)" has the meaning known in the art. Also known as bowel cancer and colon cancer, it refers to the development of cancer from the colon or rectum (parts of the large intestine). A cancer is the abnormal growth of cells that have the ability to invade or spread to other parts of the body. Signs and symptoms may include blood in the stool, a change in bowel movements, weight loss, and feeling tired all the time.

In particular, in the context of the present invention, the CRC is a microbiota-induced CRC.

Patient" can be used as well as the term "subject" and refers to a warm-blooded animal such as a mammal, in particular a human, male or female, unless otherwise specified, which is afflicted with, or has the potential to be afflicted with CRC.

"Transforming Growth Factor BetaRII receptor (TGFβRII)" has the meaning known in the art. It is a 70/80kDa TGF beta receptor.

"Group 3 Innate Lymphoid Cells (ILC3)" has the meaning known in the art. Innate lymphoid cells (ILCs) are a group of innate immune cells derived from common lymphoid progenitor (CLP) and belong to the lymphoid lineage. ILC3s can produce IL-22. "Interleukin 22 (IL-22)" has the meaning known in the art. IL-22 is an α-helical cytokine. It is a member of a group of cytokines called the IL-10 family or IL-10 superfamily (including IL-19, IL-20, IL-24, and IL-26), a class of potent mediators of cellular inflammatory responses.

"T cells" has the meaning known in the art. T lymphocytes are composed of both CD8 and CD4 T lymphocytes and play a key role in adaptive immune response.

"CD4 T lymphocytes" has the meaning known in the art. The T helper cells (Tₕ cells) are a type of T cell that plays an important role in the immune system, particularly in the adaptive immune system. They help the activity of other immune cells by releasing T cell cytokines. These cells help suppress or regulate immune responses.

Tₕ cells express the surface protein CD4 and are referred to as CD4⁺ T cells "Interleukin 17 (IL-17A)" has the meaning known in the art. Interleukin 17 is a pro-inflammatory cytokine produced by T-helper cells and is induced by IL-23.

"CD8 T lymphocytes" has the meaning known in the art. CD8+ (cytotoxic) CD8+ T cells (often called cytotoxic T lymphocytes, or CTLs) are very important for immune defense against intracellular pathogens, including viruses and bacteria, and for tumour surveillance.

"IFN-gamma" has the meaning known in the art. It is a dimerized soluble cytokine that is the only member of the type II class of interferons.

"Intestinal commensal bacteria" or "gut flora" or "gut microbiota" or "gastrointestinal microbiota" or "microbiota" has the meaning known in the art. It is the complex community of microorganisms that live in the digestive tracts of humans and other animals, including insects. The gut metagenome is the aggregate of all the genomes of gut microbiota. The gut is one niche that human microbiota inhabit. More specifically, microbiota" refers to an "ecological community of commensal, symbiotic and pathogenic microorganisms" found in and on all multicellular organisms studied to date from plants to animals. A microbiota includes bacteria, archaea, protists, fungi and viruses. In the context of the present invention, microbiota is from a warm-blooded animal such as a mammal, in particular a human, male or female. The human microbiota includes bacteria, fungi, archaea and viruses.

"Risk" in the context of the present invention, relates to the probability that an event will occur over a specific time period, as in the conversion to CRC, and can mean a patient's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a patient compared either to the absolute risks of low risk cohorts or an average population risk, which can vary by how clinical risk factors are assessed. Odds ratios, the proportion of positive events to negative events for a given test result, are also commonly used (odds are according to the formula p/(I-p) where p is the probability of event and (1- p) is the probability of no event) to no- conversion.

As used herein, the term "sample" means a substance of biological origin. In the context of the present invention, it includes blood samples and biopsy. As used herein "blood" includes whole blood, plasma, serum, circulating epithelial cells, constituents, or any derivative of blood. As used herein "biopsy" is a biopsy of colorectal sample cells or tissues. The biological sample according to the invention may be obtained from the subject by any appropriate means of sampling known from the person skilled in the art.

As used herein, "a control value" can be relative to a number or value derived from population studies, including without limitation, such patients having similar body mass index, total cholesterol levels, LDUHDL levels, systolic or diastolic blood pressure, patients of the same or similar age range, patients in the same or similar ethnic group. Such control values can be derived from statistical analyses. In one embodiment of the present invention, the control value is derived from the level of the II-17 and/or IFN-gamma in a control sample derived from one or more patients who were not subjected to CRC. Furthermore, retrospective measurement of the level of II-17 and/or IFN-gamma in properly banked historical patient samples may be used in establishing this control value. Typically, the levels of IL-17 and/or IFN-gamma in a patient who is at risk of having or developing CRC is deemed to be higher than the reference value obtained from the general population or from healthy patients. In some embodiments, a probability score is calculated, which is derived from running a model that include the levels for each IL-17 and/or IFN-gamma and optionally various patient parameters (e.g., age, gender, weight, race, cirrhosis severity). In some embodiments, the score may be established by a computer program.

As used herein, "healthy" refers to a patient not having or not developing a CRC.

### Level of expression of Transforming Growth Factor BetaRII receptor (TGFβRII) on intestinal or blood Group 3 Innate Lymphoid Cells (ILC3)

This level can be measured by any suitable method known by the man skilled in the art. In particular, ILC3 bearing TGFβRII level is measured through flow-cytometry. More particularly, it is measured through flow-cytometry obtained after labeling with a detectable antibody binding to TGFβRII.

Such kind of antibodies is well known by a man skilled in the art. Mention can for example be made of anti hTGF-bRII polyclonal goat Ile24-Asp159.

As used herein, the term "labelled", with regard to the antibody, is intended to encompass direct labelling of the antibody by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody, as well as indirect labelling of the probe or antibody by reactivity with a detectable substance. An antibody of the invention may be labelled with a radioactive molecule by any method known in the art. For example radioactive molecules include but are not limited radioactive atom for scintigraphic studies such as 1123, 1124, In111, Re186, Re188. Preferably, the antibodies are already conjugated to a fluorophore (e.g. FITC-conjugated and/or PE-conjugated).

The aforementioned assays may involve the binding of the binding partners (i.e. antibodies) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Beads may be made of different materials, including but not limited to glass, plastic, polystyrene, and acrylic. In addition, the beads are preferably fluorescently labelled.

According to the invention, methods of flow cytometry are a preferred method for determining ILC3 bearing TGFβRII level. This kind of method is well known by a man skilled in the art. Typically, a fluorescence activated cell sorting (FACS) method can be used.

### Percentage of ILC3 producing Interleukin 22 (IL-22)

This level can be measured by any suitable method known by the man skilled in the art. In particular, the percentage of IL-22 produced by ILC3 is measured through flow-cytometry. More particularly, said level is measured through flow-cytometry obtained after labeling with anti-IL-22 antibody.

Such kind of antibodies is well known by a man skilled in the art. Mention can be for example made of anti hIL-22 Mouse IgG₁ Clone # 142928.

As used herein, the term "labelled", with regard to the antibody, is intended to encompass direct labelling of the antibody by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody, as well as indirect labelling of the probe or antibody by reactivity with a detectable substance. An antibody of the invention may be labelled with a radioactive molecule by any method known in the art. For example radioactive molecules include but are not limited radioactive atom for scintigraphic studies such as I123, I124, In111, Re186, Re188. Preferably, the antibodies are already conjugated to a fluorophore (e.g. FITC-conjugated and/or PE-conjugated).

The aforementioned assays may involve the binding of the binding partners (i.e. antibodies) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Beads may be made of different materials, including but not limited to glass, plastic, polystyrene, and acrylic. In addition, the beads are preferably fluorescently libelled.

According to the invention, methods of flow cytometry are a preferred method for determining the percentage of IL-22 produced by ILC3. This kind of method is well known by a man skilled in the art. Typically, a fluorescence activated cell sorting (FACS) method can be used.

### Intestinal or blood percentage of CD4 T lymphocytes producing Interleukin 17 (IL-17A)

This level can be measured by any suitable method known by the man skilled in the art. In particular, the percentage of IL-17 produced by CD4 T cells is measured through flow-cytometry. More particularly, the percentage of IL-17 produced by CD4 T cells is measured through flow-cytometry obtained after labeling with anti-IL-17A antibody.

Such kind of antibodies is well known by a man skilled in the art. Mention can for example be made of anti hIL-17 mouse IgG1 eBio64DEC17.

As used herein, the term "labelled", with regard to the antibody, is intended to encompass direct labelling of the antibody by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody, as well as indirect labelling of the probe or antibody by reactivity with a detectable substance. An antibody of the invention may be labelled with a radioactive molecule by any method known in the art. For example radioactive molecules include but are not limited radioactive atom for scintigraphic studies such as I123, I124, In111, Re186, Re188. Preferably, the antibodies are already conjugated to a fluorophore (e.g. FITC-conjugated and/or PE-conjugated).

The aforementioned assays may involve the binding of the binding partners (i.e. antibodies) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Beads may be made of different materials, including but not limited to glass, plastic, polystyrene, and acrylic. In addition, the beads are preferably fluorescently libelled.

According to the invention, methods of flow cytometry are a preferred method for determining the percentage of IL-17 produced by CD4 T cells. This kind of method is well known by a man skilled in the art. Typically, a fluorescence activated cell sorting (FACS) method can be used.

### Ratio between the level TGFβRII expression on ILC3 and the percentage of IL-17 producing CD4 T cells

The ratio R can be calculated as follows:
R= (MFI of TGFβRII on ILC3) / (% of CD4 T cells producing IL-17)
wherein MFI has the meaning known in the art "Mean Fluorescence Intensity". Values are obtained by flow cytometry analysis.

### Ratio between the percentage of ILC3 producing IL-22 and the intestinal or blood percentage of CD4 T lymphocytes producing IL-17A

The ratio R can be calculated as follows:
R= (% of ILC3 producing IL-22) / (% of CD4 T cells producing IL-17). Values are obtained by flow cytometry analysis.

### Activation of T lymphocytes by the intestinal commensal bacteria

Activation of T lymphocytes by the intestinal commensal bacteria is measured by any suitable method known by the man skilled in the art.

As mentioned herein, "activation of T lymphocytes" corresponds to a level of expression of IL-17 and/or IFN-gamma higher than a control value.

These levels can be measured by any method any suitable method known by the man skilled in the art. In particular, it can be measured through flow-cytometry. More particularly, the percentage of IL-17 is measured through flow-cytometry obtained after labeling with anti-IL-17A antibody and the percentage of IFN-gamma through flow-cytometry obtained after labeling with anti IFN-gamma antibody. Such kind of antibodies is well known by a man skilled in the art. Mention can for example be made of anti hIL-17 mouse IgG1 eBio64DEC17 and anti hIFN-gamma Mouse IgG1, B27.

Alternatively, it can be measured with Enzyme-Linked ImmunoSpot (ELISPOT). For example, membrane surfaces in a 96-well-membrane microtiter plate are coated with capture antibody that binds a specific epitope of the cytokine being assayed. During the cell incubation and stimulation step, as the antigen-specific cells are activated, they release the cytokine, which is captured directly on the membrane surface by the immobilized antibody. The cytokine is thus captured in the area directly surrounding the secreting cell, before it has a chance to diffuse into the culture media, or to be degraded by proteases and bound by receptors on bystander cells. Subsequent detection steps visualize the immobilized cytokine; essentially the secretory footprint of the activated cell. In both cases, patient peripheral blood cells are cultured for 3-5 days with inactivated bacteria from the patient feces in culture medium conditions known by the man skilled in the art, including cell culture medium, controlled CO₂% and temperature. Patient feces are suspended in salt solution, which could include phosphate-buffered saline solution, and centrifuged at 50G for 10 minutes. The supernatant is than centrifuged at 8000 G for 5 minutes. Bacteria pellet is then inactivated by either sonication method, or heat exposure 60 for 10min, or 3 freeze-thaw cycles (-80°C).

As used herein, the term "labelled", with regard to the antibody, is intended to encompass direct labelling of the antibody by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody, as well as indirect labelling of the probe or antibody by reactivity with a detectable substance. An antibody of the invention may be labelled with a radioactive molecule by any method known in the art. For example radioactive molecules include but are not limited radioactive atom for scintigraphic studies such as I123, I124, In111, Re186, Re188. Preferably, the antibodies are already conjugated to a fluorophore (e.g. FITC-conjugated and/or PE-conjugated).

The aforementioned assays may involve the binding of the binding partners (i.e. antibodies) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Beads may be made of different materials, including but not limited to glass, plastic, polystyrene, and acrylic. In addition, the beads are preferably fluorescently libelled.

A level is considered to be statistically higher if the level of IL-17 and/or IFN-gamma in the sample of the subject is increased to above the normal level of IL-17 and/or IFN-gamma.

### Method for selecting a patient who will most likely benefit from a preventive treatment with an antibiotic, in particular Vancomycin and/or with injections of recombinant human IL-22 and/or human anti-IL-17A antibody

In the context of the method described herein, any suitable antibiotic, with activity against gram +and or gram- bacteria can be used by a man skilled in the art. Examples such as Vancomycin, Ampicillin, Metronidazole and Neomycin can be cited. These antibiotics are known by a man skilled in the art and are commercially available.

In particular, Vancomycin is used. Vancomycin is a well-known antibiotic used to treat a number of bacterial infections. It is for example recommended intravenously as a first-line treatment for complicated skin infections, bloodstream infections, endocarditis, bone and joint infections, and meningitis caused by methicillin-resistant *S*. *aureus.* Blood levels may be measured to determine the correct dose.

In the context of the present invention, the identification of the subjects who are in need of treatment of herein-described CRC is conducted as above mentioned. A clinician skilled in the art can readily identify, by the above mentioned techniques, those subjects who are in need of such treatment.

Recombinant human IL-22 are in particular those currently used and commercially available. Mention can for example be made of Recombinant human IL-22 F-.

Human anti-IL17A antibodies are in particular those currently used and commercially available. Mention can for example be made of Secukinumab.

A therapeutically effective amount of either antibiotic, in particular such as Vancomycin, recombinant human IL-22 and human anti-IL-17A antibody can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances .

As used herein, a "therapeutically effective amount" refers to an amount which is effective in reducing, eliminating, treating or controlling the symptoms of the herein-described CRC. The term "controlling" is intended to refer to all processes wherein there may be a slowing, interrupting, arresting, or stopping of the progression of the CRC described herein, but does not necessarily indicate a total elimination of all disease and condition symptoms, and is intended to include prophylactic treatment and chronic use.

The amount required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

It can be formulated into pharmaceutical compositions by admixture with one or more pharmaceutically acceptable excipients.

As used herein, a "pharmaceutically acceptable excipient" refers to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations, in particular for intravenous or oral administration, include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

### Kits

Yet another object of the invention relates to a kit for performing the method of the invention comprising means for quantifying the level of expression of Transforming Growth Factor BetaRII receptor (TGFβRII) on intestinal or blood Group 3 Innate Lymphoid Cells (ILC3) or the percentage of ILC3 producing Interleukin 22 (IL-22), and the Intestinal or blood percentage of CD4 T lymphocytes producing Interleukin 17 (IL-17A) or the activation of T lymphocytes by the intestinal commensal bacteriain in a blood sample or biopsy obtained from said patient. The kit may include a set of antibodies as above described. In a particular embodiment, the antibody or set of antibodies are labelled as above described. The kit may also contain other suitably packaged reagents and materials needed for the particular detection protocol, including solid-phase matrices, if applicable, and standards.

The invention will be further illustrated by the following figures and examples.

### FIGURES

**Figure 1****:** CRC induction by microbiota in the absence of TGF-b controls in ILC3 involves IL17 and is repressed by IL-22
**Figure 2****:** Colonic ILC3 from CRC patients express less AhR and IL-22 concomitantly with the over-representation of TH17 cells
**Figure 3****:** ROC curve illustrating the specificity of the method according to the invention to detect patients with CRC susceptibility
**Figure 4****:** Graph illustrating the number of cells producing IL-17A or IFN-gamma from duplicate after 5 days of culture. Data from one CRC and one control donor n=1.

### EXAMPLES

### Example 1

### Materials and methods

### Mice

*CD4-Cre;tgfβr2*^{*flox*/*flox*} (TGFβRII^{KO}), *CD4-Cre;tif1*-γ^{*flox*/*flox*} (TIF1-γ^{KO}), *CD4-Cre;smad4*^{*flox*/*flox*} (SMAD4^{KO}) and *CD4-Cre;tgfβr2*^{*flox*/*flox*} mice were generated as previously described (Marie et al., 2006) (Havenar-Daughton et al., 2012) *CD4-Cre;tif1*-γ^{*flox*/}*^{flox}; smad4*^{*flox*/}*^{flox} (DKO)* mice were obtained by crossing *CD4-Cre;tif*-γ^{*flox*/*flox*} and *CD4-Cre;smad4*^{*flox*/*flox*} on C57BL/6J background. RAG^{KO}*;Rorc-Cre;tif1*-γ^{*flox*/}*^{flox};smad4*^{*flox*/}*^{flox} (ILC3^{DKO})* on C57BU6J background were obtained by breedings with Rorc-Cre mice (Eberl and Littman, 2004) with *tif1-*γ^{*flox*/}*^{flox};smad4*^{*flox*/*flox*} mice. C57BL/6J mice and RAG2^{KO} (RAG^{KO}) mice were purchased from Charles River Laboratories (L'arbresle, France). Animals were maintained in AniCan, a specific pathogen free (SPF) animal facility of the Centre Léon Bérard, Lyon, France or in the Plateau de Biology Experimentale de la souris (Q3 zone), a conventional mouse facility, as well as in a SPF zone, ENS Jacques Monod, Gerland, France. The experiments were performed in accordance with the animal care guidelines of the European Union and French laws and were validated by the local Animal Ethic Evaluation Committee (CECCAPP). Unless mentioned otherwise, 6-9 weeks old mice were used, prior transformation occurring. No difference linked to animal gender was observed.

### Human samples

Human colon endoscopic biopsies were obtained from the Biological Resource Center of Centre Léon Bérard (French agreement number: AC-2013-1871) after approval by the institutional review board and ethics committee, with fully informed patient consent. CRC patients were bearing high grade dysplasia or/and adenocarcinoma in the colon, control donor were without significant histological alterations. All underwent biopsies during endoscopic check-up. Peripheral blood mononuclear cells (PBMC) were purified using standard procedure. 10ml whole blood were collected under Ethylenediaminetetraacetic acid (EDTA) and placed under Ficoll solution before centrifugation at 500G during 20 minutes at 20°C.

The cohort included 45-65 year old males and females with similar distribution in both arms. Patients were not treated with antibiotics or under chemotherapy during the 4 months preceding the biopsies. Immune cells from human biopsies were analyzed like colonic mouse cells.

### Flow cytometry

Surface staining of mouse cells was performed using the following fluorescent-conjugated antibodies: CD3 FITC (145-2C11; BD biosciences), CD5 FITC (53-7.3; BD biosciences), CD8a FITC (53.6.7; BD biosciences), B220 FITC (RA3-6B2; BD biosciences), CD19 percpCy5.5(ID3; BD biosciences), NK1.1 FITC (PK136; BD biosciences), CD11c FITC (HL3; BD biosciences), CD45 APCCy7 (30-F11; BD biosciences), CD90.2 PeCy7 (53-2.1; BD biosciences), Streptavidin PE (BD biosciences), IL-23R PE (078-1208, BD Pharmingen), CD11b FITC (M1170; ebioscience), TCRγδ FITC (UC7-13D5; ebioscience), CD4 BV711 (RM4-5; Biolegend), TGFβRII biotin (R&D systems). For intracellular cytokine staining, mouse cells were restimulated *ex vivo* for 4h with 500ng/ml PMA (Sigma Aldrich) and 500ng/ml ionomycin (Sigma Aldrich) in presence of brefeldin A (BD pharmingen) and for IL-22 staining 50ng/ml of recombinant mouse IL-23 (R&D systems) into the 4h culture. Cells were permeabilized using Foxp3 staining kit (ebioscience) according to manufacturer's protocol and intracellular staining was performed with the following antibodies: IL-22 APC (IL22JOP; BD biosciences), AhR PE (4MEJJ; ebioscience), RORγt percp-eFluor710 (B2D; ebioscience), IL-17 A700 (TC11-18H10;BD biosciences), IFN-gamma APC (XMG1.2; BD Biosciences), c-Maf eFluor 660 (sym0F1; ebioscience), SMAD4 PE (B-8; Santa Cruz) and TIF1-γ (H-106; Santa Cruz). Surface staining of human cells was performed using the following fluorescent-conjugated antibodies: CD4 BV421 (RPA-T4), CD45 BV711 (HI30), CD127 BV510 (HIL-7R-M21), CD8a APC (RPA-T8), CD11c APC (B-ly6), CD19 APC (HIB19), CD11b (ICRF44), CD14 APC-Cy7 (MφP9), CD5 APC-Cy7 (UCHT2) were purchased from BD, CD3 APC-Cy7 (SK7, ebioscience) and hTGF-βRII PE (R&D systems). For intracellular staining, cells were restimulated *ex vivo* for 4h with 500ng/ml PMA, 500ng/ml ionomycin, brefeldin A and 50ng/ml of recombinant human IL-23 (R&D systems) in the presence or not of 10ng/ml hTGFb1 (R&D systems). Intracellular staining was performed with the following antibodies: IL-17A FITC (eBio64DEC17), IL-22 (22-URTI), RORγt percp-eFluor710 (B2D; eBioscience), AhR Pe-Cy7 (FF3399), all obtained from eBioscience. Flow cytometric analysises were performed on BD Fortessa and analysis of cells was done using FlowJo software (Miltenyi biotec).

### Bone marrow transfer and adoptive T cell transfer

Bone marrow cells (BM) were isolated and depleted of CD3⁺ T lymphocytes using anti-CD3 beads (Miltenyi biotec). 10⁶ cells were injected intravenously into irradiated (6 Gray) RAG^{KO} or RAG^{KO}DKO mice. Mice were sacrificed 5 to 8 weeks after BM transfer. 10⁶ T lymphocytes (CD3⁺, Foxp3⁻) from mLN, were injected intravenously into either RAG^{KO} or RAG^{KO} DKO mice. Recipients were sacrificed 6-7 weeks after T cell transfer.

### In vivo treatments

Mice received 1µg of recombinant mouse IL-22 (R&D systems) or 20 µg of neutralizing anti-IL-17A and F mAb (R&D systems) twice a week during 4 weeks. Mice were injected 5 days before T cell transfer.

### Statistics

Unless mentioned otherwise two-tailed Student's t test was used to calculated statistical significance. P values <0.05 were considered significant. * P<0.05, ** P<0.01, *** P<0.001. Statistics including Receiver Operating Characteristics (ROC) curve were performed using Prism Software. Mann Whitney test * P<0.05, ** P<0.01.

### Results

CRC induction by microbiota in the absence of TGF-b controls in ILC3 involved IL17 and is repressed by IL-22.

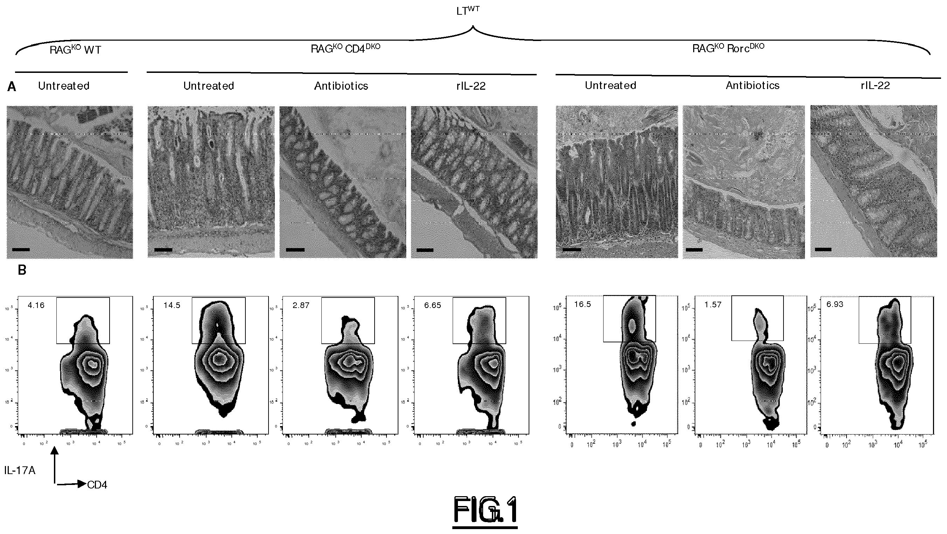

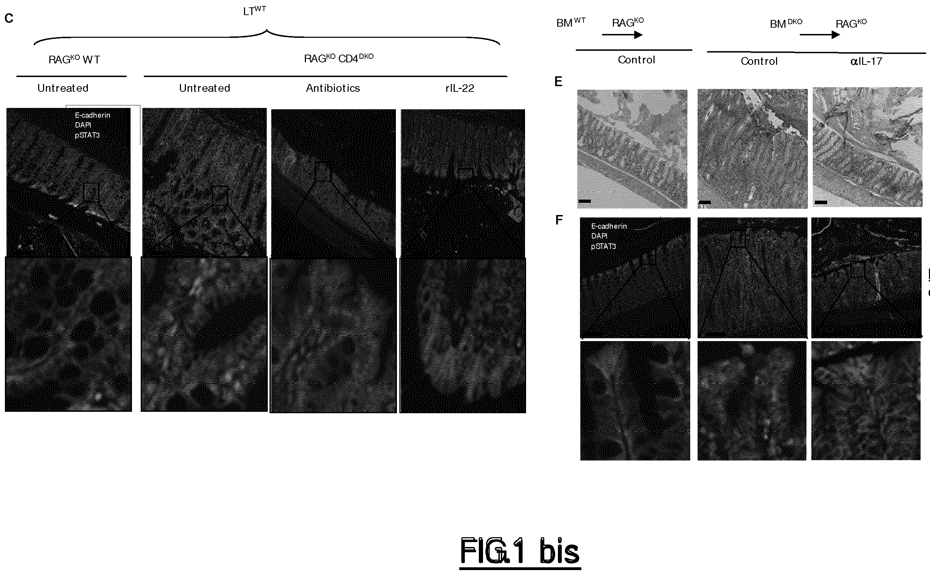

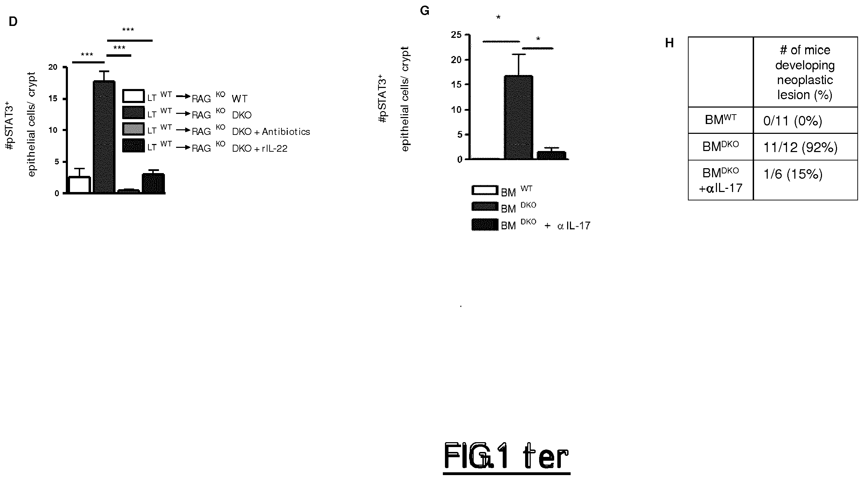

It was found that reconstitution of the T cell-compartment of mice whose ILC3 are deficient for SMAD4 and TIF1-γ, two main actors of TGF-B signaling pathways with TGF-β signaling sufficient T lymphocytes (T cells^{WT}) was adequate to induce both inflammation and transformation of the colon and of the anorectal junction, (Figure 1A). Remarkably, antibiotic treatment or rIL-22 injection of RAG^{KO}DKO and RAG^{KO}Rorc^{DKO} recipient mice was sufficient to fully prevent colonic inflammation and dysplasia (Figure 1A), suggesting that spontaneous CRC is induced by T lymphocytes in response to commensal bacteria entry into the Lamina propria (LP), due to the impairment of TGF-β controls on IL-22 production by ILC3. The analysis of transferred wild type T cells evolving in mice with ILC3 deficient for TGF-β signaling revealed a 4-fold increase in colonic TH17 cells, producing IL-17A compared to RAG^{KO} mice (Figure 1B), without affecting their IL-22 expression, similar to that observed in DKO mice. This exacerbated presence of TH17 cells in colonic LP was dependent on IL-22 and commensal bacteria. Similar to rIL-22 injection, antibiotic treatment was sufficient to abrogate the exacerbated TH17 differentiation of T cells^{WT} transferred in mice with ILC3 deficient in SMAD4 and TIF1-γ (Figure 1B). Interestingly, in DKO mice the enrichment of the colonic LP in TH17 cells preceded tumor development, suggesting that TH17 cells contribute to tumor induction in CRC. In agreement with this scenario, CRC of mice with ILC3 deprived in both SMAD4 and TIF1-γ and reconstituted with wild type T cell exhibited phosphorylation of STAT-3 in (Figure 1C-D), a known molecular link between inflammation and cancer previously reported to be indirectly activated by IL-17 in enterocytes. Remarkably, treatments with either rIL-22, stopped five days before tissue analysis, or antibiotics totally prevented STAT-3 pathway activation in CRC (Figure 1C-D). Moreover, injection of neutralizing anti-IL-17 antibody fully prevented the exacerbated activation of the STAT-3 pathway, as well as dysplasia in *Smad4* and *Tif1-γ* sufficient colonic epithelium underlining the role of TH17 cells in the spontaneous colonic transformation (Figure 1E-H). Altogether these data revealed an unexpected role for ILC3 in the prevention of CRC that is mainly orchestrated by the ability of TGF-β signaling to sustain adequate levels IL-22 production in ILC3 to prevent T cells from evolving in a bacteria-enriched LP leading to increased IL-17 production and the induction of CRC transformation.

### Colonic ILC3 from CRC patients express less IL-22 concomitantly with the over-representation of TH17 cells

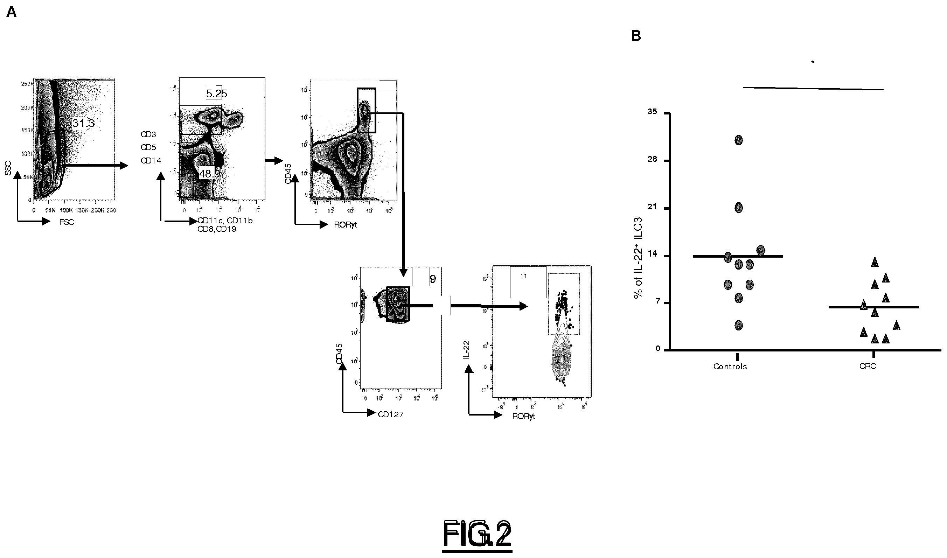

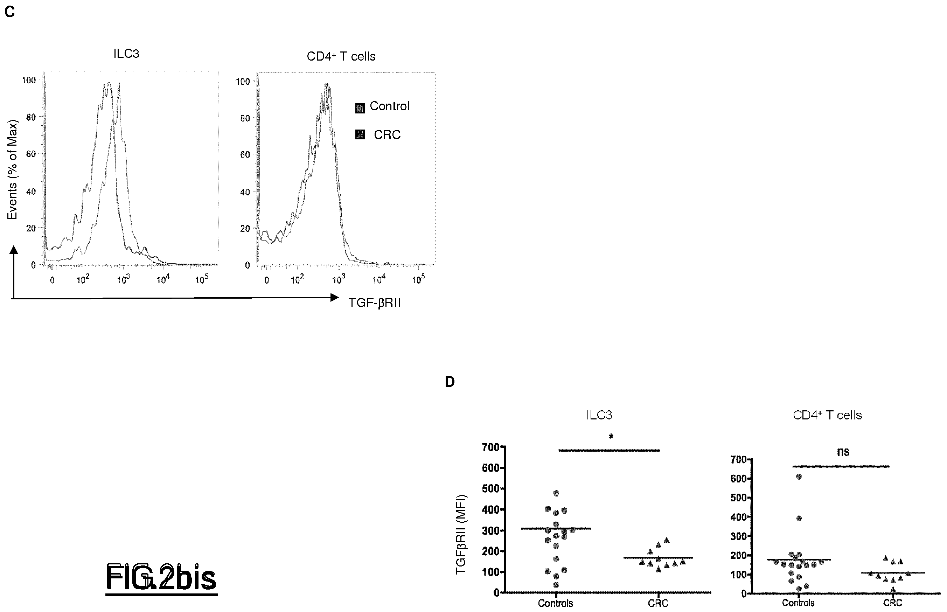

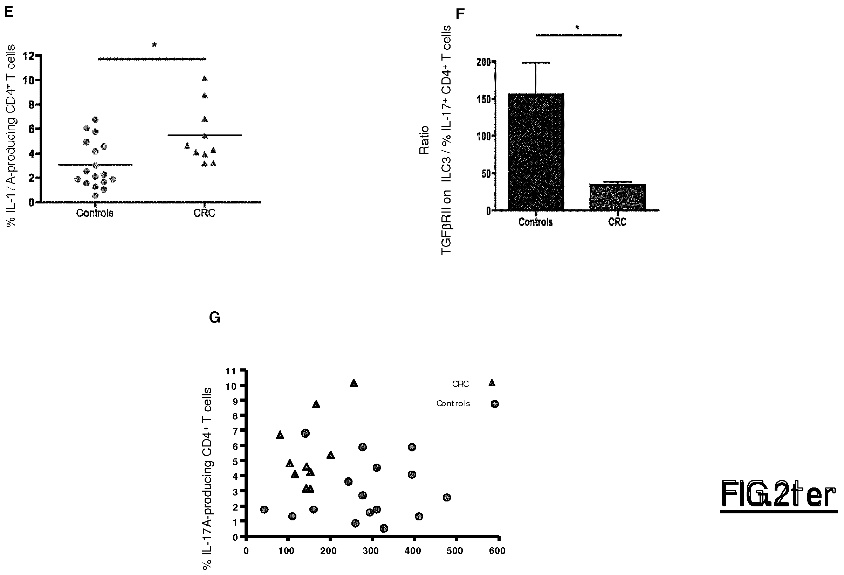

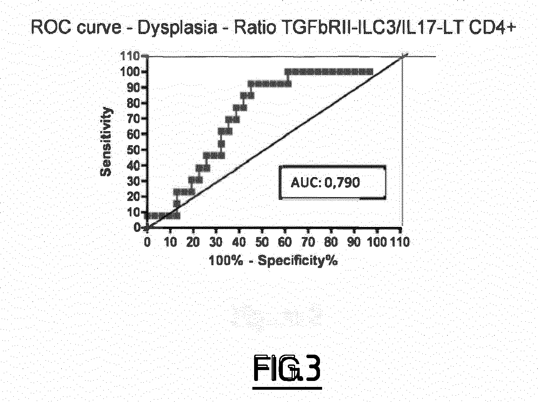

Flow cytometry analysis of fresh colon biopsies from CRC patients and controls were performed. All biopsies were performed in non-transformed areas and T cells and ILC3 analyzed by flow cytometry (Figure 2A). In line with the observations in mice, it was found that the percentage of colonic ILC3 producing IL-22 was halved in CRC patients compared to the control group (Figure 2B). Of note, the inability of colonic ILC3 from CRC patients to respond to TGF-β was associated with low levels of TGF-βR expression at their surface compared to colonic ILC3 from controls (Figure C-D). In addition, similarly to mice, it was observed that the weaken expression of ILC3 from CRC patients was concomitant with an exacerbated production of IL-17A by colonic CD4⁺ T lymphocytes (Figure E-G), whose TGF-βR surface expression remained unaffected (Figure C-D). Thus, the non-transformed area of CRC patients, exhibit similar characteristics it was found in mice with spontaneous CRC induced by the microbiota in absence of TGF-β control, suggesting that these findings in mice have direct relevance to the human pathology. It is notable that similar observations were observed on peripheral blood cells (PBMC) from CRC patients or control donors (Figure 3).

Percentage of TH17 and ILC3 sensitivity to TGF-β can predict the probability to develop CRC.

The aforementioned observations suggested that the ratio TGF-β expression on ILC3 and under the percentage of TH17 can be used to discriminate patients with a risk or not of CRC. In line with this, Receiver Operating Characteristics (ROC) curve obtained with the ratio value as the following: R= (MFI of TGFβRII on ILC3) / (% of CD4 T cells producing IL-17) for 44 patients a revealed a good specificity of the test. Moreover, the ROC curve value defined 85 as the value of ratio above which 100% of the patients can be regarded as no risk of CRC and below which 70% of the patients have risk to or already developed CRC (Figure 3).

### Example 2

### Materials and methods

### Peripheral mononuclear Blood cell (PBMC)isolation.

10ml whole blood from patients were collected under EDTA and placed under Ficoll solution before centrifugation at 500G during 20 minutes, at 20°C.

### Feces bacteria preparation

Fresh feces from control donor were resuspended in PBS solution, and filtrated under tissue to remove large debris, before centrifugation. After a first centrifugation at 50G during 10 minutes to remove smaller debris, the supernatant was next centrifuged for 5 minutes at 8000 G. The bacteria pellet was then resuspended in 5ml PBS and placed a 4°C for sonication.

### Bacteria and PBMC co-culture assay

5 µl of sonicated bacteria extract were placed on 10⁵ PBMC in 96 standard Elispot wells and centrifuged 2 minutes at 500G. Culture medium was composed of completed RMPI 1640, hepes, and penicillin streptomycin according to classic procedure. 20UI of recombinant human IL-2 were added and plates were incubated at 37°C 5% CO₂ for 5 days.

### Elispot

Elispot for inflammatory cytokines such as IL-17A IFN-gamma, TNF-a GM-CSF were revealed according vendor instructions.

### Results

### Analysis of the PBMC reactivity against feces bacteria

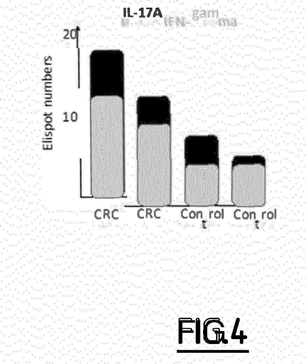

It was analyzed whether PBMCS from patients with CRC can react against feces bacteria antigens. As illustrated in Figure 4, in contrast to control PBMC donors, PBMC from CRC patients produced more IL-17A and IFN-gamma in response to bacteria antigens revealing that the reactivity of PBMC to fecal bacteria antigen is a feature of CRC patients.

## Claims

1. A method for determining if a patient is at risk of having or developing a colorectal cancer (CRC), comprising the step of measuring in a biopsy or blood sample from said patient:
(a)the level of expression of Transforming Growth Factor BetaRII receptor (TGFβRII) on Intestinal or blood Group 3 Innate Lymphoid Cells (ILC3) and the intestinal or blood percentage of CD4 T lymphocytes producing Interleukin 17 (IL-17A); or
(b) the percentage of ILC3 producing Interleukin 22 (IL-22) and the intestinal or blood percentage of CD4 T lymphocytes producing Interleukin 17 (IL-17A); or
(c)the activation of T lymphocytes by the intestinal commensal bacteria.

2. The method of claim 1, comprising the step of measuring in a biopsy or blood sample from said patient:
(a) the level of ILC3 bearing TGFβRII and the percentage of IL-17 produced by CD4 T cells.

3. The method of claim 1 or 2, wherein ILC3 bearing TGFβRII level is measured through flow-cytometry.

4. The method of claim 1, comprising the step of measuring in a biopsy or blood sample from said patient:
(b) the percentage of IL-22 produced by ILC3 and the percentage of IL-17 produced by CD4 T cells.

5. The method of claim 1 or 4, wherein the percentage of IL-22 produced by ILC3 is measured through flow-cytometry.

6. The method according to any of the preceding claims, wherein the percentage of IL-17 produced by CD4 T cells is measured through flow-cytometry.

7. The method according to any of claims 1-3 and 6, wherein a ratio between the level TGFβRII expression on ILC3 and the percentage of IL-17 producing CD4 T cells below 85 is indicative of a patient at risk of having or developing a CRC.

8. The method according to any of claims 1 and 4-6, wherein a ratio between the percentage of ILC3 producing IL-22 and the intestinal or blood percentage of CD4 T lymphocytes producing IL-17A below 0.5 is indicative of a patient at risk of having or developing a CRC.

9. The method of claim 1, comprising the step of measuring in a biopsy or blood sample from said patient:
(c) the activation of T lymphocytes by the intestinal commensal bacteria.

10. The method according to any of claims 1 or 9, wherein the activation of T cells by the intestinal commensal bacteria is measured by comparing the level of expression of IL-17 and/or IFN-gamma to a control value.

11. The method according to any of claims 1, 9 or 10, wherein an activation of T cells by the intestinal commensal bacteria is indicative of a patient at risk of having or developing a CRC.

12. The method according to claim 11, wherein a level of expression of IL-17 and/or IFN-gamma higher than the control value indicates the activation of T cells by the intestinal commensal bacteria.

13. A method for selecting a patient who will most likely benefit from a preventive treatment with an antibiotic, in particular Vancomycin and/or with injections of recombinant human IL-22 and/or human anti-IL-17A antibody, wherein said method comprises determining if a patient is at risk of developing a colorectal cancer by the method according to any of the preceding claims.
